# EUROPEAN PATENT SPECIFICATION

(11) **EP 1 357 952 B1**
(45) Date of publication and mention of the grant of the patent: **01.09.2010**
(21) Application number: 02720846.1
(22) Date of filing: 23.01.2002
(51) Int. Cl.: A61L 27/34

(54) **METHOD FOR COATING MEDICAL DEVICE SURFACES**
VERFAHREN ZUR BESCHICHTIGUNG VON MEDIZINISCHEN GERÄTEN
PROCEDE DE REVETEMENT POUR SURFACES DE DISPOSITIFS MEDICAUX

(30) Priority: 31.01.2001 US 265370 P
(43) Date of publication of application: 05.11.2003
(73) Proprietor: Medtronic, Inc., Minneapolis, MN 55432-5604 (US)
(72) Inventor: KEOUGH, James, R., Maplewood, MN 55109 (US); TRESCONY, Paul, V., Champlin, MN 55316 (US); VERHOEVEN, Michel, NL-6221 BD Maastricht (NL); KOULIK, Edouard, NL-6227 ET Maastricht (NL)
(74) Representative: Golding, Louise Ann
(86) International application number: PCT/US2002/002120
(87) International publication number: WO 2002/060505

(56) References cited:
- EP-A- 0 596 615
- US-A- 4 642 242
- US-A- 5 928 916
- US-A- 6 033 719
- IKADA Y: "SURFACE MODIFICATION OF POLYMERS FOR MEDICAL APPLICATIONS" BIOMATERIALS, ELSEVIER SCIENCE PUBLISHERS BV., BARKING, GB, vol. 15, no. 10, 1 August 1994 (1994-08-01), pages 725-736, XP000464803 ISSN: 0142-9612

## Description

### Background of the Invention

For many years, a number of medical devices (e.g., pacemakers, vascular grafts, stents, heart valves, artificial hearts, heart pumps, hip prostheses, heart lung machines, catheters, kidney dialysis equipment, etc.) that contact bodily tissue or fluids of living persons or animals have been developed, manufactured, and used clinically. A major problem with such articles is that their surfaces tend to adsorb a layer of proteins from tissues and fluids such as tears, urine, lymph fluid, blood, blood products, and other fluids and solids derived from blood. The composition and organization of the adsorbed protein layer is dependent upon fluid flow rate and the surface properties of the device material including roughness, hydrophobicity and chemical composition. It is believed that the adsorbed protein layer influences, if not controls, further biological reactions (e.g., platelet adherence and activation, blood coagulation, and complement activation). Implantable medical devices also tend to serve as foci for infection of the body by a number of bacterial species. These device-associated infections are promoted by the tendency of these organisms to adhere to and colonize the surface of the device. Consequently, it has been of great interest to physicians and the medical industry to develop surfaces that are less prone in promoting the adverse biological reactions such as thrombosis, inflammation and infection that typically accompany the implantation of a medical device.

Proteins in blood are compact, very complex structures which possess charged groups, hydrogen bonding groups, and nonpolar hydrophobic groups. Proteins are polypeptides made up of amino acid residues. A protein comprising two or more polypeptide chains is called an oligomeric protein. Proteins in blood tend to possess a net surface charge due to the internalization of their nonpolar groups, or residues, thus enabling them to remain soluble. Upon contact with a hydrophobic surface, a protein may undergo a conformational change, or unfolding. The unfolding of the protein allows for hydrophobic interactions to occur between the protein's nonpolar residues and the foreign surface. The strength of these interactions is governed by the hydrophobicity of the surface and the protein. During the adsorption process, the interactions between the surface and the protein cause the displacement of water molecules that reside between the soluble protein and the surface. The displacement of water molecules strengthens the hydrophobic interactions between the surface and protein, causing further unfolding of the protein. As the protein continues to unfold, its solubility decreases, thereby reducing the tendency for the unfolded protein to desorb. During the adsorption process, the degree of unfolding a protein undergoes may or may not alter its physiological properties. If a protein has lost its physiological properties, the protein is termed "denatured." Denatured proteins adsorbed onto a medical device surface tend to be irreversibly bound.

It has been shown that hydrophilic surfaces demonstrate a rapid and extensive exchange of proteins, whereas hydrophobic surfaces demonstrate a much slower and less extensive exchange of proteins. In general, the adsorption of proteins onto hydrophobic surfaces tends to be irreversible, while the adsorption of proteins onto hydrophilic surfaces tends to be reversible.

There has been great interest in developing biomaterials that possess protein-resistant surfaces for use in medical devices. To this end, various techniques have been attempted to create materials with hydrophilic surfaces. For example, a medical device coating comprising ∞, ω-aldehyde terminated poly(ethylene oxide) which is cross-linked to the device via exposure to a high energy source is disclosed in U.S. Pat. No. 5,507,804 (Llanos) and 5,645,882 (Llanos). The high energy source results in the formation of free radicals, ions, electrons, protons, neutrons, alpha particles, beta particles, gamma radiation, X-ray radiation or ultraviolet radiation. An amine (e.g., n-heptyl amine) is applied to the substrate (i.e., an intraocular lens) via plasma deposition. After treatment with the amine, the substrate surface containing amine groups is reacted with aldehyde functionalized endcapped poly(ethylene oxide) in the presence of a reducing agent (e.g., sodium cyanoborohydride).

U.S. Pat. No. 4,459,317 (Lambert) and U.S. Pat. No. 4,487,808 (Lambert) disclose a two-step process for applying a solution containing isocyanate, evaporating the solvent, applying a second solution containing poly(ethylene oxide) and then evaporating the solvent of the second solution. The final coating is then cured via heating. U.S. Pat. No. 4,585,666 (Lambert) and U.S. Pat. No. 4,666,437 (Lambert) disclose a two-step process for applying a solution containing isocyanate, evaporating the solvent, applying a second solution containing poly(N-vinylpyrrolidone) and an amine catalyst. The solvent is then evaporated and the coating is cured via heating.

U.S Pat. No. 4,906,237 (Johansson et al.) discloses a method to enhance a hydrophilic coating by applying a solution of an osmolality increasing compound such as glucose, sorbitol, sodium chloride, sodium citrate, sodium benzoate, calcium chloride, potassium chloride, potassium iodide and potassium nitrate to a non-reactive hydrophilic polymer surface layer and then evaporating the solvent of the solution. U.S. Pat. No. 4,589,873 (Schwartz et al.) discloses a method of contacting a substrate with a solution of a hydrophilic polymer, e.g., poly(N-vinylpyrrolidone), in a solvent, e.g., dimethylformamide, and heating the substrate to evaporate the solvent. U.S. Pat. No. 4,990,357 (Karakelle et al.) discloses a lubricious hydrophilic coating comprising a hydrophilic polymer, e.g., polyacrylic acid, polyvinyl pyridine, polyvinyl methyl ether, polyhydroxyethyl methacrylate, poly(ethylene oxide) and poly(N-vinylpyrrolidone, and a hydrophilic polyetherurethane in a solvent. Upon coating of the substrate, the solvent is removed via heating to a temperature between 50 and 200°C.

U.S. Pat. No. 5,061,424 (Karimi et al.) discloses a composition which includes poly(N-vinylpyrrolidone) and a polyurethane that is melt processed (extruded or molded) to give a shaped article. The article's surface becomes lubricious when contacted with water. U.S. Pat. No. 5,084,315 (Karimi et al.) discloses a lubricious coating with a composition which adheres to the base polymer. This patent also discloses a method in which the base polymer and the coating composition are coextruded so that a layer of the coating composition is laminated onto the base polymer. The coating composition contains at least two and, preferably, three or more components. The first component is a hydrophilic lubricating polymer which provides lubricity to the coated article when wet. The lubricating polymer may be any extrudable polymer which adsorbs water and migrates to the surface of the coating composition. The second component of the composition is a polymeric matrix material which serves as a carrier for the lubricating polymer and as a binder to provide adherence of the coating composition to the base polymer.

U.S. Pat. No. 4,657,820 (Halpern et al.) discloses applying an acrylic polymer solution (hydroxyethylmethacrylate) to a plastic object and letting the solution dry as an anchor coat. Then applying, as a top coat, an aqueous solution containing 1% sodium hyaluronate and between 0.1 and 15% albumin. U.S. Pat. No. 4,801,475 (Halpern et al.) discloses a method for first coating a plastic surface with an aqueous solution of a mucopolysaccharide, i.e., hyaluronate. Then precipitating the mucopolysaccharide onto a surface using a water-miscible solvent, e.g., acetone, methyl alcohol, methyl ethyl ketone and ethyl alcohol. The mucopolysaccharide is then crosslinked and immobilized onto the surface by applying a solution of catalyzed (with dibutyltin dilaurate) organic-soluble aliphatic polyisocyanate, i.e., Desmodur N. U.S. Pat. Nos. 5,037,677 (Halpern et al.) and 5,023,114 (Halpern et al.) disclose a method for coating an object with a solution containing a solvent and a polymer, e.g., ethylmethacrylate and isocyanatoethyl methacrylate. Following coating, the solvent is removed, thereby forming a film. A second aqueous solution of sodium hyaluronate is then applied, followed by the removal of water, thereby forming a film. Lastly, the first and second films are chemically joined together via heating.

U.S. Pat. No. 5,643,681 (Voorhees et al.) discloses coating materials comprising triblock copolymers having a polysiloxane block flanked by polylactone blocks. The coating can be applied by dipping, spraying, or passage through a coating bath. U.S. Pat. No. 5,041,100 (Rowland et al.) discloses catheters which possess a hydrophilic lubricious coating comprising a mixture of polyurethane and poly(ethylene oxide). The coating solution is applied as an aqueous emulsion via dipping or spraying, followed by drying. U.S. Pat. No. 5,077,352 (Elton) discloses a one-step coating process for applying a mixture of an isocyanate, a polyol, poly(ethylene oxide), and a solvent to a substrate surface. Following coating, the solvent is evaporated, and the mixture is cured via heating, thereby forming a polyurethane coating which contains poly(ethylene oxide). U.S. Pat. No. 5,160,790 (Elton) discloses a one-step process for applying a mixture of an isocyanate, a polyol, poly(N-vinylpyrrolidone), and a solvent to a substrate surface. Following coating, the solvent is evaporated, and the mixture is cured via heating, thereby forming a polyurethane coating which contains poly(N-vinylpyrrolidone).

U.S. Pat. No. 5,272,012 (Opolski) discloses a process for applying a solution of a urethane, a slip additive and, optionally, a crosslinking agent. A high MW, hard, non-yellowing, water based urethane is preferred. Preferred slip additives include silicones and siloxanes, fluorochemicals, poly(N-vinylpyrrolidone) copolymers and a variety of waxes. Preferred crosslinking agents include aziridine and carbodiimide. A primer layer may be included to enhance the adhesion between the substrate and the coating. A preferred primer is ethylene acrylic acid. Following application, the coating is cured via heating.

U.S Pat. No. 4,100,309 (Micklus et al.) discloses a two-step coating method. First, a polyisocyanate containing prepolymer and polyurethane solution is applied to the substrate and allowed to dry, thereby forming an anchor coat. Next, a poly(N-vinylpyrrolidone) solution is applied, thereby forming a top coat. The coating is again dried to form a poly(N-vinylpyrrolidone)-polyurethane coating. U.S. Pat. No. 4,642,267 (Creasy et al.) discloses a polymer blend consisting of a polyurethane and poly(N-vinylpyrrolidone) in a solvent. The polymer blend is then used as a substrate or as a coating. U.S. Pat. No. 4,847,324 (Creasy) discloses a polymer blend consisting of polyvinylbutyral and poly(N-vinylpyrrolidone) in a solvent. The polymer blend is then used as a substrate or as a coating. U.S. Pat. No. 4,373,009 (Winn) discloses a two-step coating process. First, a polyisocyanate solution is applied to a substrate and the solvent is evaporated. Next, a hydrophilic copolymer solution is applied and allow to dry and cure at room temperature or at an elevated temperature.

U.S. Pat. No. 5,576,072 (Hostettler et al.) and U.S. Pat. No. 5,662,960 (Hostettler et al.) disclose cohesive lubricious polyurethane-polyurea hydrogel coatings which are covalently bonded to plasma-treated polymeric substrates, or chemically-treated metallic substrates, and which further contain at least one additional hydrogel polymer, having a dissimilar composition to the polyurethane-polyurea hydrogel, to form a commingled hydrogel network. Polymeric substrates are first treated with a nitrogen containing plasma, thereby forming amino groups on the surface. Metallic substrates are first treated with aminosilane primers. The resulting surfaces are then coated with the hydrophilic polyurethane-polyurea hydrogel forming prepolymer containing terminal isocyanate groups to affix the permanently bonded polyurethane-polyurea first coat. At least one water soluble hydrogel polymer containing isocyanate reactive groups is then applied as a dilute solution to convert the polyurethane-polyurea prepolymer intermediate to a hydrogel polymer.

U.S. Pat. No. 5,001,109 (Whitbourne) and U.S. Pat. No. 5,331,027 (Whitbourne) discloses a lubricious coating comprising a hydrophilic polymer, e.g. poly(N-vinylpyrrolidone), and a stabilizing polymer, e.g., cellulose ester. The method of applying the coating includes first exposure of the substrate to a solution of the stabilizing polymer followed by evaporation of the solvent at an elevated temperature. Second, applying the coated substrate to a solution of the hydrophilic polymer and evaporating the solvent at elevated temperature. U.S. Pat. No. 4,973,493 (Guire), U.S. Pat. No. 4,979,959 (Guire) and U.S. Pat. No. 5,263,992 (Guire) disclose a method for attaching a hydrophilic polymer with a linking compound possessing a photochemically reactive group capable, upon activation, of covalently bonding to a solid surface, and possessing a different reactive group, capable of binding to the molecules of the hydrophilic polymer. The method comprises reacting the linking compound with the hydrophilic polymer, followed by photochemically reacting the linking compound, attached to the hydrophilic polymer, to the solid surface. U.S. Pat. No. 5,258,041 (Guire et al.) and U.S. Pat. No. 5,217,492 (Guire et al.) disclose a technique for attaching biomolecules to substrates using spacers which comprise a hydrophilic chain carrying a hydrophobic group, derived from an aminoalkyl carboxylic acid, capable of becoming embedded in the hydrophobic surface, and a stopping group, being hydrophilic and positioned between the bulk of the spacers hydrophilic chain and the hydrophobic guiding group. The spacer is covalently attached to the surface via a latent reactive group adjacent the guiding group.

U.S. Pat. No. 5,670,558 (Onishi et al.) discloses a lubricious coating process which comprises coating the substrate with a solution containing a water-soluble or water-swellable block or graft copolymer having a reactive functional group consisting of an epoxy, acid chloride, aldehyde or isocyanate group and crosslinking the polymer to form a layer on the surface that will form a hydrogel when wetted. Upon application, the coating is cured by heating to 40°C and above. U.S. Pat. No. 5,091,205 (Fan) discloses a two-step method for preparing coated substrates by first coating the substrate with a solution of a polyisocyanate followed by drying in an oven of the primer coat. Second, applying a solution of a carboxylic acid containing polymeric top coat, e.g., poly(acrylic acid), poly(methacrylic acid) or poly(isocrotinic acid), followed by drying at a temperature between 50 and 100°C. U.S. Pat. No. 5,295,978 (Fan et al.) discloses a three-step method for preparing coated substrates by first coating the substrate with a solution of a polyisocyanate. Second, applying a solution of a carboxylic acid containing polymer, e.g., poly(acrylic acid), poly(methacrylic acid) or poly(isocrotinic acid). And third, applying a solution containing a poly(N-vinyl lactam), e.g., poly(N-vinyl pyrrolidone), or a poly(lower-alkylene oxide), e.g., poly(ethylene oxide), and, thereafter drying the coated substrate at a temperature between 50 and 100°C.

U.S. Pat. No. 5,558,900 (Fan et al.) and U.S. Pat. No. 5,645,931 (Fan et al.) disclose a one-step method for preparing coated substrates by coating the substrate with a solution of a polyisocyanate and a poly(ethylene oxide) and drying the coated substrate. U.S. Pat. No. 5,620,738 (Fan et al.) discloses either a one-step method or a two-step method for preparing coated substrates. In the two-step method, the substrate is first coated with a binder polymer and subsequently coated with a hydrophilic polymer. In the one-step method, the binder polymer and the hydrophilc polymer are applied to the substrate in a single step. The binder polymer is a copolymer made from two or more monomers (e.g., vinyl chloride and vinyl acetate) which comprise of at least one vinyl moiety and at least one carboxylic acid moiety (e.g., acrylic, methacrylic, maleic, itaconic or fumaric). The carboxylic acid groups in the binder polymer promote bonding between the binder polymer and the hydrophilic polymer.

U.S. Pat. No. 4,840,851 (Golander et al.) discloses a process for forming a lubricious coating by applying a solution containing poly(ethylene oxide), a radiation curable cross-linking agent (e.g., hexamethylenedioldiacrylate) and a solvent which is also a swelling solvent for the substrate. The poly(ethylene oxide) has one unmodified end and at least one radiation curable ethylenically unsaturated group (e.g., acrylic or methacrylic group) at the other end.

Another approach for minimizing undesirable biological reactions associated with medical devices is to attach various biomolecules to their surfaces for the attachment and growth of a cell layer which the body will accept. Biomolecules such as growth factors, cell attachment proteins, and cell attachment peptides have been used for this purpose. In addition, biomolecules such as antithrombogenics, antiplatelets, anti-inflammatories, antimicrobials, and the like have also been used to minimize adverse biomaterial-associated reactions. For example, U.S. Pat. No. 5,344,455 (Keogh et. al.), U.S. Pat. No. 5,476,509 (Keogh et. al.), U.S. Pat. No. 5,545,213 (Keogh et. al.), U.S. Pat. No. 5,821,343 (Keogh), U.S. Pat. No. 5,728,420 (Keogh), U.S. Pat. No. 5,891,506 (Keogh), U.S. Pat. No. 5,928,916 (Keogh), U.S. Pat. No. 5,945,319 (Keogh), U.S. Pat. No. 6,033,719 (Keogh), U.S. Pat. No. 6,017,741 (Keogh) and U.S. Pat. No. 5,925,552 (Keogh and Trescony) all disclose various methods for attaching biomolecules to medical device biomaterial surfaces.

Heparin has been coupled to biomaterial surfaces. Heparin, an anionic biomolecule, is of great interest to a number of investigators for the development of non-thrombogenic blood-contact biomaterial surfaces. Heparin, a sulphated glycosaminoglycan, is known to inhibit blood coagulation by binding to antithrombin III (ATIII), a serine proteinase inhibitor (serpin) found in blood. The binding of heparin to ATIII activates the serpin which, in turn, inactivates the procoagulant serine proteinases thrombin, factor Xa and, to some extent, factors IXa, XIa and XIIa. Heparin accelerates the reactions of ATIII with the blood clotting proteinases from ∼1000 to ∼10,000-fold. Surfaces bearing bound heparin have been shown to have anticoagulant activity, therefore, heparinization tends to be a popular technique for improving the thromboresistance of biomaterials.

Another approach for minimizing undesirable biological reactions associated with medical devices is to attach compounds containing sulfonate groups, similar to those which give heparin its anticoagulant activity. These surfaces are typically referred to as "heparin-like" surfaces. For example, U.S. Pat. No. 5,278,200 (Coury, et. al.) and U.S. Pat. No. 5,429,618 (Keogh) disclose heparin-like materials and coatings comprising sulfonate groups.

Current techniques for immobilization of hydrophilic polymers to material surfaces are generally limited to particular chemical groups possessed by the hydrophilic polymer and/or the material surface. Thus, there is a need for alternative methods for immobilizing hydrophilic polymers to medical device substrate surfaces.

### Summary of the Invention

The present invention has certain objects that address problems existing in the prior art with respect to surface coatings of therapeutically important products. Various embodiments of the present invention provide solutions and advantages to one or more of the problems existing in the prior art with respect to surface coatings. To each of the embodiments the present invention provides one or more particular features that is taught or further illustrated herein.

The present invention has an object of solving a number of problems associated with the use of medical devices. The present invention includes within its scope methods for attaching hydrophilic polymers to biomaterial surfaces for use in medical devices.

The present invention provides a method for making a medical device having at least one hydrophilic polymer immobilized on a biomaterial surface. The method comprises chemically binding under appropriate reaction conditions a hydrophilic polymer to a biomaterial surface. The hydrophilic polymer comprises a chemical moiety capable of forming a hydrogen bond with a catechol moiety (such as, for example, a hydroxyl moiety, a phosphate moiety, a sulfate moiety, a carboxylate moiety, an amide moiety, a guanidino moiety, or an amine moiety). The biomaterial surface comprises a catechol moiety.

The present invention provides another method for making a medical device having at least one hydrophilic polymer immobilized on a biomaterial surface. The method again comprises chemically binding under appropriate reaction conditions a hydrophilic polymer to a biomaterial surface. However, the hydrophilic polymer in this method comprises a catechol moiety and the biomaterial surface comprises a chemical moiety capable of forming a hydrogen bond with a catechol moiety (such as, for example, a hydroxyl moiety, a phosphate moiety, a sulfate moiety, a carboxylate moiety, an amide moiety, a guanidino moiety, or an amine moiety).

The present invention also provides a method for making a medical device having at least one hydrophilic polymer immobilized on a primer located on a biomaterial surface. The method comprises chemically binding under appropriate reaction conditions a hydrophilic polymer to a primer coated on a biomaterial surface. The hydrophilic polymer comprises a chemical moiety capable of forming a hydrogen bond with a catechol moiety (such as, for example, a hydroxyl moiety, a phosphate moiety, a sulfate moiety, a carboxylate moiety, an amide moiety, a guanidino moiety, or an amine moiety). The primer coated on a biomaterial surface comprises a catechol moiety.

The present invention also provides another method for making a medical device having at least one hydrophilic polymer immobilized on a primer located on a biomaterial surface. The method again comprises chemically binding under appropriate reaction conditions a hydrophilic polymer to a primer coated on a biomaterial surface. However, the hydrophilic polymer in this method comprises a catechol moiety and the primer coated on a biomaterial surface comprises a chemical moiety capable of forming a hydrogen bond with a catechol moiety (such as, for example, a hydroxyl moiety, a phosphate moiety, a sulfate moiety, a carboxylate moiety, an amide moiety, a guanidino moiety, or an amine moiety).

### Brief Description of the Drawings

The following drawings depict certain embodiments of the invention. They are illustrative only and do not limit the invention otherwise disclosed herein.
- Fig. 1:: Catechol Moieties
- Fig. 2:: Quinone Moieties
- Fig. 3:: Semiquinone Moieties
- Fig. 4:: Example of Michael Addition

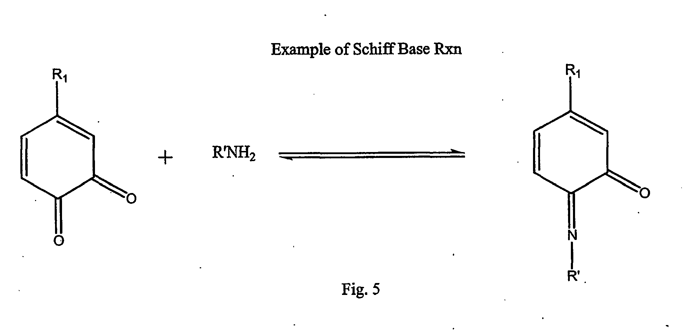

- Fig. 5:: Example of Schiff Base Rxn

### Detailed Description of the Invention

As used in the specification and claims hereof, the following terms have the particular meanings and definitions set forth below.

I define the term "chemical bond" appearing herein to be interpreted broadly to encompass not only covalent bonding and ionic bonding but also interactions, such as, for example, van der Waals forces and hydrogen bonding.

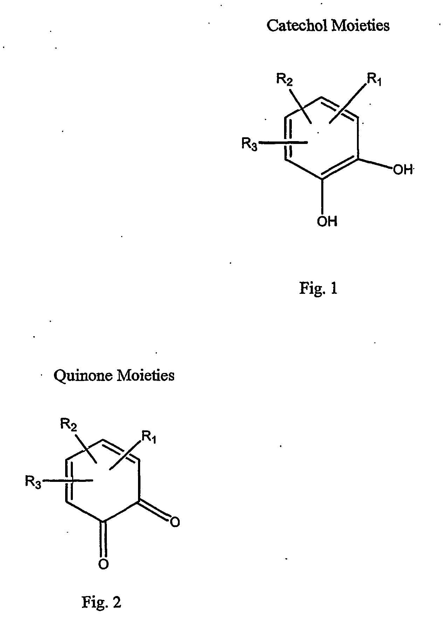

I define the term "catechol" appearing herein as a phenyl ring comprising two polar hydroxyl (-OH) groups attached to the ring of Fig. 1, wherein:
R₁ is a hydrogen, a bond to a medical device, a hydrophilic polymer or biomolecule, a substituted linear or branched (C₁-C₆)-alkyl amine; or a linear or branched (C₁-C₆)-alkyl amine;
R₂ and R₃ independently selected can be hydrogen, hydroxy, R' or OR'; R' representing optionally substituted linear or branched (C₁-C₆)-alkyl, optionally substituted linear or branched (C₂-C₆)-alkenyl, optionally substituted linear or branched (C₂-C₆)-alkynyl, optionally substituted (C₃-C₇)-cycloalkyl, optionally substituted aryl, optionally substituted biphenyl, optionally substituted heteroaryl; SO₃⁻, or PO₃⁻;
R₂ and R₃ taken together can form a 3-12 membered mono- or bicyclic ring, a hematoxylin ring, in which one or more of the carbon atoms may be substituted with nitrogen, oxgen or sulfur, wherein each of these ring systems is optionally mono or polysubstituted with halogen, C1-C6-alkyl, hydroxy, C₁-C₆ alkoxy, C₁₋₆-alkoxy, C₁-₆alkoxy-C₁-₆alkyl, nitro, amino, cyano, trifluoromethyl, C₁₋₆-monoalkyl- or dialylamino or oxo;
the term "aryl" denoting phenyl or naphthyl;
the term "heteroaryl" denoting a saturated or unsaturated, 4- to 11-membered, mono- or bi-cyclic group containing from 1 to 4 hetero atoms selected from the group nitrogen, oxygen, and sulphur;
the expression "optionally substituted" applied to the terms "alkyl", "alkenyl", "alkynyl", and "cycloalkyl" means that those groups are, if desired, substituted by one or more halogen or (C₃ -C₇)-cycloalkyl, hydroxy, linear or branched (C₁ -C₆)-alkoxy, optionally substituted aryl and/or optionally substituted heteroaryl; and the expression "optionally substituted" applied to the terms "aryl", "biphenyl", and "heteroaryl" means that those groups are, if desired, substituted by one or more halogen and/or linear or branched (C₁ -C₆)-alkyl, linear or branched (C₁ -C₆)-trihaloalkyl, hydroxy, linear or branched (C₁ -C₆)-alkoxy, nitro, amino which is optionally substituted by one or two identical or different, linear or branched (C₁-C₆)-alkyl; linear or branched (C₁-C₆)-alkylcarbonyl, cyano, carboxy, and/or aminocarbonyl optionally substituted by one or two identical or different linear or branched (C₁-C₆)-alkyl,
their enantiomers and diastereoisomers, and also pharmaceutically acceptable addition salts thereof with an acid or base..

Catechols are also known as diphenols. The hydroxyl groups are capable of forming hydrogen bonds (H-bonds). Hydrogen bonding of the hydroxyls of a catechol to hydrophilic polymers is competitive with that of water. The pair of hydroxyls of a catechol provides more than one site for hydrogen bonding giving the catechol an increased ability to form cyclic structures containing two or more hydrogen bonds with anion or hydrogen comprising compounds. For this reason, catechols are very effective at forming H-bonds. Catechols may form hydrogen bonds with other chemical groups or moieties capable of hydrogen bonding, for example, carboxylate moieties (RCOOH), amide moieties (RCONH₂), hydroxyl moieties (ROH), amine moieties (RNH₂), guanidino moieties (RNHC(NH)NH₂), sulfate moieties (RSO₃H) and phosphate moieties (ROPO₃H₂).

The catechol comprises a phenol group. Phenols are fairly acidic compounds being stronger acids than water, but considerably weaker acids than carboxylic acids. Phenols are converted into their water soluble salts, i.e., phenoxide ions, by aqueous hydroxides, but not by aqueous bicarbonates. The salts are converted into the free water insoluble phenols by aqueous mineral acids, carboxylic acids or carbonic acid. Phenoxide ions tend to be soluble in water and insoluble in organic solvents. Most phenols do not dissolve in aqueous bicarbonate solutions, since, as mentioned earlier, phenols are liberated from their salts by the action of carbonic acid.

The holding of a hydrogen or metal atom between two atoms of a single molecule is called chelation. Catechols have the ability to chelate metallic ions or metal oxides present at a metal surface. For this reason, catechols can form organometallic complexes at the surface of metal that are not easily desorbed. Catechols have been shown to form complexes with Al(III), Fe(III), Si(IV) and many others in mono-, bis-, and tris-complexes of extreme stability.

Pyrocatechase, a dioxygenase, catalyzes the opening of the catechol ring by reaction with O₂, thereby forming a product that contains two carboxyl groups. Catechol oxidase catalyzes the conversion of a catechol to a quinone. Catechol oxidase is interchangeably known as tyrosinase, catecholase, polyphenoloxidase, phenoloxidase, and phenolase. Mushroom and bacterial tyrosinases have been used *in vitro* to modify catechols to quinones. Mushroom tyrosinase has a long history of use as an agent of site-directed modification. Other oxidative agents that may be used alone or in combination to convert a catechol to a quinone may include without limitation oxygen (O₂), Fe₃⁺, hydrogen peroxide (H₂O₂) and sodium periodate (NaIO₄). Quinones may be reduced back to catechols by, for example, ascorbate. The term "catechol" appearing herein may mean any one or more of a catechol alone or a combination of different catechols.

Catechol is oxidizable with periodate, which may be provided as periodic acid or salts thereof, such as sodium periodate, potassium periodate, or other alkali metal periodates. Typically, a stoichiometric amount of periodate is used to oxidize the desired number of catechol moieties to form quinone moieties, however less than a stoichiometric amount or more than a stoichiometric amount may be used.

Periodate oxidation of a catechol moiety is generally carried out in an aqueous solution, preferably an aqueous buffered solution, at a temperature that does not destroy the desired properties of the material. Generally, buffers having a pH in a range between about 4 and about 9 can be used, with a pH between about 6 and about 8 desired for certain pH sensitive materials. Generally, the oxidation is carried out at a temperature between about 0 and about 50 degrees Celsius, and preferably at a temperature between about 4 and about 37 degrees Celsius. Depending on the material, oxidation reactions can be carried out for as short as a few minutes to as long as many days. Commonly, oxidation is complete within 24 hours. Long-term oxidation reactions are preferably performed in the dark to prevent "overoxidation."

Treatment times and temperatures for the oxidation process tend to be inversely related. That is, higher treatment temperatures require relatively shorter treatment times. Time and temperature limitations of the present invention are generally governed by the stability of the materials imparted by the oxidation process. Wide latitude may be employed in determining the optimum conditions for a particular system. Such conditions may be determined readily by one skilled in the art by routine experimentation upon examination of the information presented herein.

Subsequent to oxidation, the reaction solution may be stored prior to use at about 4 degrees Celsius. Typically, the storage stability of the reaction solution at a neutral pH or slightly acidic pH may extend between about one and about fourteen days and sometimes even months when stored in the dark.

I define the term "quinone" appearing herein as a ring structure comprising two carbonyl groups of Fig. 2, wherein:
R₁ is a hydrogen, a bond to a medical device, a hydrophilic polymer or biomolecule, a substituted linear or branched (C₁-C₆)-alkyl amine; or a linear or branched (C₁-C₆)-alkyl amine;
R₂ and R₃ independently selected can be hydrogen, hydroxy, R' or OR'; R' representing optionally substituted linear or branched (C₁-C₆)-alkyl, optionally substituted linear or branched (C₂-C₆)-alkenyl, optionally substituted linear or branched (C₂-C₆)-alkynyl, optionally substituted (C₃-C₇)-cycloalkyl, optionally substituted aryl, optionally substituted biphenyl, optionally substituted heteroaryl; SO₃⁻, or PO₃⁻;
R₂ and R₃ taken together can form a 3-12 membered mono- or bicyclic ring, a hematoxylin ring, in which one or more of the carbon atoms may be substituted with nitrogen, oxgen or sulfur, wherein each of these ring systems is optionally mono or polysubstituted with halogen, C1-C6-alkyl, hydroxy, C₁-C₆ alkoxy, C₁₋₆-alkoxy, C₁₋₆alkoxy-C₁₋₆alkyl, nitro, amino, cyano, trifluoromethyl, C₁₋₆-monoalkyl- or dialylamino or oxo;
the term "aryl" denoting phenyl or naphthyl;
the term "heteroaryl" denoting a saturated or unsaturated, 4- to 11-membered, mono- or bi-cyclic group containing from 1 to 4 hetero atoms selected from the group nitrogen, oxygen, and sulphur;
the expression "optionally substituted" applied to the terms "alkyl", "alkenyl", "alkynyl", and "cycloalkyl" means that those groups are, if desired, substituted by one or more halogen or (C₃ -C₇)-cycloalkyl, hydroxy, linear or branched (C₁ -C₆)-alkoxy, optionally substituted aryl and/or optionally substituted heteroaryl; and the expression "optionally substituted" applied to the terms "aryl", "biphenyl", and "heteroaryl" means that those groups are, if desired, substituted by one or more halogen and/or linear or branched (C₁ -C₆)-alkyl, linear or branched (C₁ -C₆)-trihaloalkyl, hydroxy, linear or branched (C₁ -C₆)-alkoxy, nitro, amino which is optionally substituted by one or two identical or different, linear or branched (C₁-C₆)-alkyl; linear or branched (C₁-C₆)-alkylcarbonyl, cyano, carboxy, and/or aminocarbonyl optionally substituted by one or two identical or different linear or branched (C₁-C₆)-alkyl,
their enantiomers and diastereoisomers, and also pharmaceutically acceptable addition salts thereof with an acid or base..
A carbonyl group contains a carbon-oxygen double bond (C=O). Quinones are cyclic unsaturated ketones. Quinones are generally colored. The term "quinone" appearing herein may mean any one or more of a quinone alone or a combination of different quinones. Quinones may form Michael-type adducts by the addition of nucleophiles to unsaturated ketones. Chemical groups that may undergo a Michael Addition reaction with quinones include without limitation amine groups, sulfhydryl groups and hydroxyl groups. Further, quinones may form Schiff bases involving nucleophilic attack of the ketyl group by an amine. Reaction of a quinone with a guanidino moiety may form a 5-membered ring.

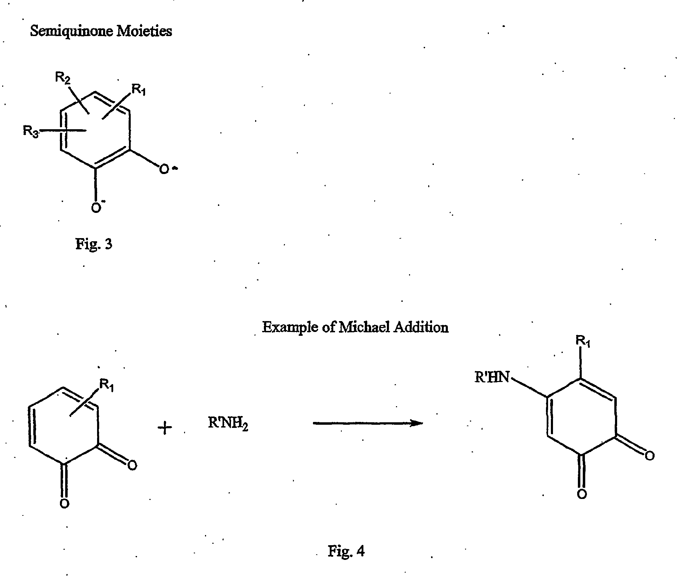

One-electron reduction of a quinone creates a semiquinone radical, i.e., a free radical. A semiquinone radical can also be formed by a comproportionation reaction between a quinone and a catechol. I define the term "semiquinone" appearing herein as a ring structure comprising two oxygen groups of Fig. 3, wherein:
R₁ is a hydrogen, a bond to a medical device, a hydrophilic polymer or biomolecule, a substituted linear or branched (C₁-C₆)-alkyl amine; or a linear or branched (C₁-C₆)-alkyl amine;
R₂ and R₃ independently selected can be hydrogen, hydroxy, R' or OR'; R' representing optionally substituted linear or branched (C₁-C₆)-alkyl, optionally substituted linear or branched (C₂-C₆)-alkenyl, optionally substituted linear or branched (C₂-C₆)-alkynyl, optionally substituted (C₃-C₇)-cycloalkyl, optionally substituted aryl, optionally substituted biphenyl, optionally substituted heteroaryl; SO₃⁻, or PO₃⁻;
R₂ and R₃ taken together can form a 3-12 membered mono- or bicyclic ring, a hematoxylin ring, in which one or more of the carbon atoms may be substituted with nitrogen, oxgen or sulfur, wherein each of these ring systems is optionally mono or polysubstituted with halogen, C1-C6-alkyl, hydroxy, C₁-C₆ alkoxy, C₁₋₆-alkoxy, C₁₋₆alkoxy-C₁₋₆alkyl, nitro, amino, cyano, trifluoromethyl, C₁₋₆-monoalkyl- or dialylamino or oxo;
the term "aryl" denoting phenyl or naphthyl;
the term "heteroaryl" denoting a saturated or unsaturated, 4- to 11-membered, mono- or bi-cyclic group containing from 1 to 4 hetero atoms selected from the group nitrogen, oxygen, and sulphur;
the expression "optionally substituted" applied to the terms "alkyl", "alkenyl", "alkynyl", and "cycloalkyl" means that those groups are, if desired, substituted by one or more halogen or (C₃ -C₇)-cycloalkyl, hydroxy, linear or branched (C₁ -C₆)-alkoxy, optionally substituted aryl and/or optionally substituted heteroaryl; and the expression "optionally substituted" applied to the terms "aryl", "biphenyl", and "heteroaryl" means that those groups are, if desired, substituted by one or more halogen and/or linear or branched (C₁ -C₆)-alkyl, linear or branched (C₁ -C₆)-trihaloalkyl, hydroxy, linear or branched (C₁ -C₆)-alkoxy, nitro, amino which is optionally substituted by one or two identical or different, linear or branched (C₁-C₆)-alkyl; linear or branched (C₁-C₆)-alkylcarbonyl, cyano, carboxy, and/or aminocarbonyl optionally substituted by one or two identical or different linear or branched (C₁-C₆)-alkyl,
their enantiomers and diastereoisomers, and also pharmaceutically acceptable addition salts thereof with an acid or base..

A quinone moiety may react chemically with a primary amine moiety to form a relatively unstable imine moiety (R'N=CHR). The reaction of a quinone moiety with a primary amine moiety thereby forming an imine moiety is commonly referred to as a Schiff base reaction. The Schiff base reaction may be carried out in an aqueous solution, preferably an aqueous buffered solution, at a temperature that does not destroy the desired properties of the material. Generally, buffers having a pH in a range between about 6 and about 10 can be used, with a pH between about 6 and about 8 desired for certain pH sensitive materials. Generally, the Schiff base reaction is carried out at a temperature between about 0 and about 50 degrees Celsius, and preferably at a temperature between about 4 and about 37 degrees Celsius. Depending on the material, the Schiff base reaction can be carried out for as short as a few minutes to as long as many hours.

To stabilize the relatively unstable imine linkage, subsequent reductive alkylation of the imine moiety is carried out using reducing agents (i.e., stabilizing agents) such as, for example, sodium borohydride, sodium cyanoborohydride, and amine boranes, to form a secondary amine (R'NH-CH₂R). This reaction can also be carried out under the same conditions as described above for the Schiff base reaction.

Treatment times and temperatures for most reactions tend to be inversely related. That is, higher treatment temperatures require relatively shorter treatment times. Time and temperature limitations of the present invention are generally governed by the stability of the materials imparted by the reaction. Wide latitude may be employed in determining the optimum conditions for a particular system. Such conditions may be determined readily by one skilled in the art by routine experimentation upon examination of the information presented herein.

I define the term "hydrophilic polymer" appearing herein as a water-soluble or water-swellable polymer or copolymer comprising one or more hydrophilic chemical groups or moieties. Examples of hydrophilic chemical groups include, but are not limited to, chemical groups capable of forming hydrogen bonds, for example, carboxylate groups, amide groups, hydroxyl groups, amine groups, guanidino groups, sulfate groups and phosphate groups. Hydrophilic polymers with high molecular weight generally exhibit some degree of lubricity on hydration. For this reason, hydrophilic polymers may be used to reduce friction on the surfaces of medical devices. Preferred hydrophilic polymers may generally have a molecular weight (MW) between about 100,000 and about 2,000,000.

Hydrophilic polymers may be polymerized from or comprising, for example, acrylamide monomers, methacrylamide monomers, 2-acrylamido-2-methylpropane sulfonic acid (AMPS), acrylic acid, N-(3-aminopropyl) methacrylamide hydrochloride, N-vinylpyrrolidone, polyethylene oxide (PEO), saccharides or glycans such as hyaluronic acid or chondroitin sulfate. Other examples of hydrophilic polymers include poly(alkylene oxalate), poly(vinyl alcohol), ionene (ionic amine) polymers, caprolactone copolymers, chitin and its derivatives, agarose, cellulosic derivatives, poly(maleic anhydride) and polysaccharides. Hydrophilic polymers may be a naturally occurring or chemically synthesized.

Polyacrylamide is a hydrophilic polymer comprising amide groups which may be hydrolyzed, thereby forming carboxyl groups in the side chains of the polyacrylamide polymer backbone. Primary amines may also be formed in side chains of the polymer backbone via a Hofmann Rearrangement reaction. Acrylamide monomers may be copolymerized with a number of different monomers thereby producing acrylamide copolymers comprising various reactive chemical groups. For example, acrylamide may be copolymerized with 2-acrylamido-2-methylpropane sulfonic acid (AMPS) to produce a hydrophilic polymer comprising both amide groups and sulfate groups. Acrylamide may also be copolymerized with acrylic acid to produce a hydrophilic polymer comprising both amide groups and carboxyl groups. Acrylamide may also be copolymerized with N-(3-aminopropyl) methacrylamide hydrochloride to produce a hydrophilic polymer comprising both amide groups and amine groups.

Polyvinylpyrrolidone is a hydrophilic polymer comprising tertiary amide groups which may be hydrolyzed, thereby forming secondary amine groups and carboxyl groups in the side chains of the PVP polymer backbone. Similar to acrylamide, the monomer N-vinylpyrrolidone may be copolymerized with a number of different monomers thereby producing PVP polymers comprising a variety of reactive groups. In addition, PVP may be made to be end functionalized with various reactive chemical groups, e.g., hydroxyl groups.

Polyethylene oxide (PEO) is another hydrophilic polymer. PEO polymers may be chemically functionalized at the ends of the PEO polymer backbone to comprise various reactive chemical groups, e.g., amine groups or hydroxyl groups.

Polysaccharides or glycans such as hyaluronic acid are other examples of hydrophilic polymers. In general, polysaccharides comprise hydroxyl groups. In addition, polysaccharides generally may be oxidized to comprise reactive aldehyde groups.

Hydrophilic polymers suitable for use in the present invention comprise a chemical moiety capable of forming a hydrogen bond with a catechol moiety (such as, for example, a hydroxyl moiety, a phosphate moiety, a sulfate moiety, a carboxylate moiety, an amide moiety, a guanidino moiety, or an amine moiety), optionally in combination with one or more moieties selected from a chemical moiety capable of forming a covalent chemical bond with a quinone moiety (such as, for example, an amine moiety, a sulfhydryl moiety, a hydroxyl moiety or a guanidino moiety), and a chemical moiety capable of forming a chemical bond with a semiquinone radical. Additionally, hydrophilic polymers suitable for use in the present invention may comprise a catechol moiety optionally in combination with one or more of the chemical moieties mentioned. Further, the term "hydrophilic polymer" appearing herein may mean any one or more of a hydrophilic polymer alone or a combination of different hydrophilic polymers. Hydrophilic polymers that do not comprise one or more chemical moieties mentioned above may be furnished with any one of them by chemical modification through a number of methods well known in the art.

Coating thickness may be one of the most important parameters in producing "slippery" hydrophilic surfaces. In general, a slippery hydrophilic coating needs to be at least a few microns in thickness to achieve the physical property of slip. To achieve an appropriate thickness, multiple manufacturing steps may be required. In addition to multiple coating steps, the hydrophilic polymers used in the coating process may need to be of considerable molecular weight, i.e. hundreds of thousands to millions. Besides coating thickness, desirable features of a slippery coating may include branching, cross-linking and/or mechanical interlocking of the coating with the device surface.

I define the term "biomaterial" appearing herein as a material that is substantially insoluble in human or animal bodily fluids and that is designed and constructed to be placed in or onto the body or to contact fluid of the body. Ideally, a biomaterial will not induce undesirable reactions in the body such as blood clotting, tissue death, tumor formation, allergic reaction, foreign body reaction (rejection) or inflammatory reaction; will have the physical properties such as strength, elasticity, permeability and flexibility required to function for the intended purpose; may be purified, fabricated and sterilized easily; will substantially maintain its physical properties and function during the time that it remains implanted in or in contact with the body. Biomaterials suitable for use in the present invention comprise a chemical moiety capable of forming a hydrogen bond with a catechol moiety (such as, for example, a hydroxyl moiety, a phosphate moiety, a sulfate moiety, a carboxylate moiety, an amide moiety, a guanidino moiety or an amine moiety), optionally in combination with one or more moieties selected from a chemical moiety capable of being chelated by a catechol moiety (such as, for example, metallic ions or metal oxides), a chemical moiety capable of forming a covalent chemical bond with a quinone moiety (such as, for example, an amine moiety, a sulfhydryl moiety, a hydroxyl moiety or a guanidino moiety), and a chemical moiety capable of forming a chemical bond with a semiquinone radical. Additionally, biomaterials suitable for use in the present invention may comprise a catechol moiety optionally in combination with one or more of the chemical moieties mentioned. Further, the term "biomaterial" appearing herein may mean any one or more of a biomaterial alone or a combination of different biomaterials. Biomaterials that do not comprise one or more chemical moieties mentioned above may be furnished with any one of them by chemical modification through a number of methods well known in the art.

Biomaterials or substrates that may be used according to one method of the present invention include metals such as titanium, titanium alloys, TiNi alloys, shape memory alloys, super elastic alloys, aluminum oxide, platinum, platinum alloys, stainless steels, stainless steel alloys, MP35N, elgiloy, haynes 25, stellite, pyrolytic carbon, silver carbon, glassy carbon, polymers such as polyamides, polycarbonates, polyethers, polyesters, polyolefins including polyethylenes or polypropylenes, polystyrenes, polyurethanes, polyvinylchlorides, polyvinylpyrrolidones, silicone elastomers, fluoropolymers, polyacrylates, polyisoprenes, polytetrafluoroethylenes, rubber, minerals or ceramics such as hydroxapatite, human or animal protein or tissue such as bone, skin, teeth, collagen, laminin, elastin or fibrin, organic materials such as wood, cellulose, or compressed carbon, and other materials such as glass, and the like. Biomaterials of the present invention made using these materials may be coated, or uncoated, derivatized or underivatized. It is also recognized that certain side chain can be incorporated into polymers by modifying the choose of cross-linking agent. Further additional functional groups may be introduced into the polymer during polymer synthesis to form functional groups, such as allophanate and biuret groups which may gbe used to form additional chemical bonds with the quinones, semiquinones, and catechols of the present invention.

I define the term "primer" appearing herein as an anchor or base coat which enhances adhesion between the substrate or biomaterial and the hydrophilic polymer coating. The material used in the primer coat may be very substrate specific, i.e., different primer coats may be used for different substrates. A primer may generally be applied to a biomaterial surface, i.e., medical device, from a solvent via dipping, brushing or spraying. Following application of the primer solution, the solvent is evaporated at temperatures generally above 40°C. Sometimes thermal curing of the primer coating also takes place at elevated temperatures.

A coating process comprising a primer step may be necessary for medical device surfaces that lack reactive chemical groups. The coating process may first involve coating the device surface with a base or primer coat. The primer coat may adhere, e.g., by adsorption, onto the device surface. The primer coat may consist of polymers such as polyethyleneimine (PEI) or polyallylamine or it may consist of polymers capable of forming self-assembly films on surfaces, e.g, diblock or triblock copolymers. A crosslinker such as, for example, diisocyanate, divinylsulfone, dopamine or dihydroxybenzaldehyde may also be included in the primer coat to help stabilize it on the device surface. Depending on the chemical makeup of the primer coat, it may or may not bind covalently to the device surface, however, it would comprise reactive chemical groups or moieties, e.g., hydroxyl moieties, phosphate moieties, sulfate moieties, carboxylate moieties, amide moieties, guanidino moieties, sulfhydryl moieties, amine moieties, catechol moieties or quinone moieties, capable of binding a hydrophilic polymer during one or more subsequent coating steps. In addition, various methods of depositing quinones, semiquinones, and catechol on to metal surfaces are known in the art and can be employed, such as sputtering, spraying, dipping, electrolysis, sublimation, volitization, and the like.

Primers suitable for use in the present invention comprise a chemical moiety capable of forming a hydrogen bond with a catechol moiety (such as, for example, a hydroxyl moiety, a phosphate moiety, a sulfate moiety, a carboxylate moiety, an amide moiety, a guanidino moiety, or an amine moiety), optionally in combination with one or more moieties selected from a chemical moiety capable of forming a covalent chemical bond with a quinone moiety (such as, for example, an amine moiety, a sulfhydryl moiety, a hydroxyl moiety or a guanidino moiety), and a chemical moiety capable of forming a chemical bond with a semiquinone radical. Additionally, primers suitable for use in the present invention may comprise a catechol moiety optionally in combination with one or more of the chemical moieties mentioned. Further, the term "primer" appearing herein may mean any one or more of a primer alone or a combination of different primers. Primers that do not comprise one or more chemical moieties mentioned above may be furnished with any one of them by chemical modification through a number of methods well known in the art.

PEI and polyallylamine, both examples of primers, comprise reactive amine moieties. As mentioned earlier, the hydrophilic polymer would comprise its own reactive chemical moieties, e.g., quinones, capable of bonding to the primer coat's reactive chemical groups. Besides imparting lubricious characteristics, the hydrophilic polymer may also cross-link the primer coat, thereby stabilizing it on the biomaterial surface.

Biomaterials, primers and/or hydrophilic polymers of the present invention comprising an unsubstituted amide moiety may be converted into an amine-functional material via a Hofmann rearrangement reaction, also known as a Hofmann degradation of amides reaction. For example, Wirsen et al., "Bioactive heparin surfaces from derivatization of polyacrylamide-grafted LLDPE", Biomaterials, 17, 1881-1889 (1996), demonstrated the conversion of unsubstituted amide moieties of a polyacrylamide-low density polyethylene film into primary amine moieties using a Hofmann rearrangement reaction. In another example, Sano et al., "Introduction of functional groups onto the surface of polyethylene for protein immobilization", Biomaterials, 14, 817-822 (1993), demonstrated the conversion of unsubstituted amide moieties of a polyacrylamide-high density polyethylene film into primary amine moieties using a Hofmann rearrangement reaction. In another example, Fuller et al., "A new class of amino acid based sweeteners", J. Am. Chem. Soc., 107, 5821-5822 (1985), demonstrated the conversion of an unsubstituted amide moiety of an amino acid into a primary amine moiety using a Hofmann rearrangement reaction. In another example, Loudon et al., "Conversion of aliphatic amides into amines with [I,I-bis(trifluoroacetoxy)iodo]benzene. 1. Scope of the reaction", J. Org. Chem., 49, 4272-4276 (1984), demonstrated the conversion of various unsubstituted amide moieties, including the amide side chain in a glutamine amino acid residue, into primary amine moieties using Hofmann rearrangement reactions.

The Hofmann rearrangement reaction which converts an unsubstituted amide moiety into a primary amine moiety may be carried out with chemical reactants such as, for example, bromine, bromide, bromite, hypobromite, chlorine, chloride, chlorite, hypochlorite, lead tetraacetate, benzyltrimethylammonium tribromide and hypervalent organoiodine compounds such as, for example, [bis(trifluoroacetoxy)iodo]benzene, hydroxy(tosyloxy)iodobenzene and iodosylbenzene. A general discussion of Hofmann rearrangement reactions is contained in Comprehensive Organic Synthesis, Volume 6, 800-806, Pergamon Press. For example, Kajigaeshi et al., "An efficient method for the hofmann degradation of amides by use of benzyltrimethylammonium tribromide", Chemistry Letters, 463-464 (1989), described various methods for obtaining amines from amides using the Hofmann rearrangement reaction. These methods include, for example, the use of bromine or chlorine in an alkaline solution, the use of lead tetraacetate in an alcohol solution, the use of [bis(trifluoroacetoxy)iodo]benzene in an aqueous acetonitrile solution, the use of sodium bromite in an alkaline solution, and the use of benzyltrimethylammonium tribromide in an alkaline solution. Catalysts such as, for example, triethylamine, tin(IV) chloride, dibutylstannyl dilaurate or pyridine are sometimes used in a Hofmann rearrangement reaction. Typical solvents include, for example, water, hydroxides, methoxides, alcohols, dimethylformamide, acetonitrile, benzene, carboxylic acids or combinations thereof.

Depending on the chemical reactants, the Hofmann rearrangement reaction may be carried out under acidic, neutral or basic conditions. Although applicants do not wish to be bound by any single theory, it is generally believed that when the reaction is carried out with particular amine forming agents in an aqueous base a N-halo amide intermediate is formed. An isocyanate is then formed from the N-halo amide intermediate. The formed isocyanate then readily hydrolyzes into a primary amine. In contrast, when the reaction is carried out in an alcohol a carbamate is generally formed. The carbamate may then be hydrolyzed into a primary amine. For example, when the reaction of an amide with bromine is carried out in methanol containing sodium methoxide instead of in aqueous base, the product is a carbamate which is easily converted to an amine via hydrolysis. Depending on the reaction conditions, side-reactions such as chain scission, hydrolysis and/or urea formation may occur. However, side-reactions may be minimized by changes in the reaction conditions. For example, changes in the reaction conditions such as pH, time, temperature and/or the amount of amine forming agent may minimize various side-reactions.

In general, the Hofmann rearrangement reaction is carried out with an amine forming agent in amounts ranging from about 0.5 eq. to about 2 eq. based on the amide content of the biomolecule, biomaterial, primer or hydrophilic polymer. In addition, the reaction is generally carried out at a temperature between about -10 and about 100 degrees Celsius, preferably from about 0 and about 50 degrees Celsius. Depending on the material, a Hofmann rearrangement reaction may be carried out for as short as a few minutes to as long as many hours. Time, temperature and pH limitations of the present invention are generally governed by the stability of the materials imparted by the Hofmann rearrangement process. Wide latitude may be employed in determining the optimum conditions for a particular system. Such conditions may be determined readily by one skilled in the art by routine experimentation upon examination of the information presented herein.

I define the term "amine forming agent" appearing herein to include any chemical agent or combination of chemical agents capable of forming an amine moiety upon its or their reaction with an unsubstituted amide moiety. Examples of amine forming agents include, for example, bromine, bromide, bromite, hypobromite, chlorine, chloride, chlorite, hypochlorite, lead tetraacetate, benzyltrimethylammonium tribromide and hypervalent organoiodine compounds such as, for example, [bis(trifluoroacetoxy)iodo]benzene, hydroxy(tosyloxy)iodobenzene and iodosylbenzene. Amine forming agents include any of the many possible Hofmann rearrangement reactants. As mentioned above, the term "amine forming agent" appearing herein may mean any one or more of an amine forming agent or a combination of different amine forming agents.

Biomaterials, primers and/or hydrophilic polymers of the present invention comprising, in addition to an unsubstituted amide moiety, a primary amine moiety of which is desired to be left intact and unreacted following coupling may be protected or blocked prior to the Hofmann rearrangement reaction. For example, a protein may comprise both a lysine amino acid residue and, for example, an asparagine amino acid residue. There are a number of established coupling procedures which may couple a protein comprising a lysine amino acid residue to a substrate surface through the protein's lysine amino acid residue. However, a protein's lysine amino acid residues are typically associated with the protein's biologically active site. Therefore, coupling a protein to a substrate surface via a protein's primary amine moiety in the side chain of its lysine amino acid residue may destroy the biological properties of the attached protein. However, a protein's lysine amino acid residue may be protected or blocked by a number of methods well known to those skilled in the art. For example, the amine moiety may be protected using, for example, a tert.butyloxycarbonyl (Boc) group which is typically cleaved with acid, a benzyloxycarbonyl (Z) group which is typically cleaved by hydrogenolysis, a biphenylisopropyloxycarbonyl (Bpoc) group which is typically cleaved with acid, a triphenylmethyl (trityl) group which is typically cleaved with acid, a 9-fluoroenylmethyloxycarbonyl (Fmoc) group which is typically cleaved with base or a blocking group which is pH stable but is cleaved by enzyme-catalyzed hydrolysis. The appropriate amine-blocking group to use to protect the amine moiety will depend highly on the entire sequence of reaction conditions chosen for biomolecule attachment or crosslinking. Following blocking of the amine moiety of the lysine residue, the amide moiety of the asparagine residue may then be converted into an amine moiety via a Hofmann rearrangement reaction. The protein may then be coupled to the substrate via one method of the present invention through the newly formed amine moiety. Following coupling, the amine-blocking group may then be removed, thereby preserving the protein's biological activity. This type of blocking scheme may be employed on biomaterials, primers and/or hydrophilic polymers of the present invention which contain amine moieties which are desired to be left intact and unreacted.

Biomaterials of the present invention not comprising unsubstituted amides on their surface may be amidated readily through a number of methods well known in the art. For example, unsubtituted amides may be provided by ceric ion grafting acrylamide to a biomaterial surface as set forth in U.S. Pat. No. 5,344,455 to Keogh et al. Alternatively, for example, a grafted acrylamide-containing polymer may be attached by radiation grafting as set forth in U.S. Pat. No. 3,826,678 to Hoffman et al. There are a number of surface-derivatization techniques (e.g., grafting techniques) well known in the art for creating substrate surfaces comprising unsubstituted amide moieties. For example, techniques based on ceric ion initiation, ozone exposure, corona discharge, UV irradiation and ionizing radiation (⁶⁰Co, X-rays, high energy electrons, plasma gas discharge) are known. In addition, amides can generally be prepared by reaction of ammonia with acid chlorides. This reaction is commonly known as ammonolysis. Acid chlorides are prepared by substitution of -Cl for the -OH group of a carboxylic acid. Reagents commonly used to form acid chlorides from carboxylic acids include thionyl chloride (SOCl₂), phosphorus trichloride (PCl₃) and phosphorus pentachloride (PCl₅). Two amino acids comprising carboxylic acid moieties which may be converted into acid chlorides are aspartic acid (Asp, D) amino acid and glutamic acid (Glu, E) amino acid. The acid chloride moieties may then be converted into amide moieties followed by conversion into amine moieties. In addition, treatment of an ester moiety with ammonia, generally in ethyl alcohol solution, will yield an amide moiety.

I define the term "guanidino moiety" appearing herein to include guanidine, guanidinium, guanidine derivatives such as (RNHC(NH)NHR'), monosubstituted guanidines, monoguanides, biguanides, biguanide derivatives such as (RNHC(NH)NHC(NH)NHR"), and the like. In addition, the term "guanidino moiety" appearing herein may mean any one or more of a guanide alone or a combination of different guanides.

Guanidine is the imide of urea, or the amidine of carbamic acid. It is a very strong base with a pKₐ of 13.5 in water. The great basicity of guanidine is a result of the stability of the conjugated acid (guanidinium) in water. The positive charge on the guanidinium ion can be spread equally among the three nitrogens by resonance. The guanidinium ion is also quite hydrophilic and is well solvated in aqueous media due to the extensive hydrogen bonding of six potential hydrogen bond donors to the solvent. The partial positive charge of the hydrogen bond donors increases their strength for donation to the negative dipole of water. Crystal structures of simple guanidinium derivatives have revealed several common features. First, the C-N single bond length in an alkyl guanidine is typically shorter than the usual C-N single bond length. Usually, the three C-N bonds in the guanidinium group itself are nearly equal in length with an average of 1.33 A. The three N-C-N bond angles are almost always near 120°.

The guanidinium group's features make it a very attractive moiety. For example, its high basicity (a pKₐ of 13.5 for guanidinium itself) allows it to remain protonated over a much wider range of pH than does the ammonium group. In fact, at physiological pH, all but a small fraction of the guanidine molecules will exist as positively charged species. The guanidinium group's enhanced hydrogen bonding capabilities, typically two linear hydrogen bonds, allow it to form tighter complexes with anions that are capable of hydrogen bonding. In fact, the guanidinium group may form characteristic pairs of zwitterionic hydrogen bonds which provide binding strength by their charge and structural organization by their arrangement. Another feature of guanidines are their ability to react with quinone moieties under mild alkaline conditions to form covalent bonds. The reaction of a guanidino moiety and a quinone moiety is similar to a Schiff base reaction. In some cases, it may be desirable to use a stabilizing agent such as borate ion (BO₃⁻) to stabilize the resultant compound.

Biomaterials, primers and/or hydrophilic polymers of the present invention comprising an unsubstituted amide moiety may be modified to comprise guanidino moieties. The method includes converting an unsubstituted amide moiety (RCONH₂) into an amine-functional material (RNH₂). Amine-functional biomolecules, biomaterials, primers and/or hydrophilic polymers may be modified to comprise guanidino moieties by reaction with compounds such as S-ethylthiouronium bromide, S-ethylthiouronium chloride, O-methylisourea, O-methylisouronium sulfate, O-methylisourea hydrogen sulfate, S-methylisothiourea, 2-methyl-1-nitroisourea, aminoiminomethanesulfonic acid, cyanamide, cyanoguanide, dicyandiamide, 3,5-dimethyl-1-guanylpyrazole nitrate and 3,5-dimethyl pyrazole. For example, reaction of amines with O-methylisourea, S-methylisourea, S-ethylthiouronium bromide or S-ethylthiouronium chloride, thereby yielding guanidino moieties, are generally completed after 8 hours at 70 degrees Celsius in a solution of sodium hydroxide (NaOH) at pH 10. Reactions of amines with aminoiminomethanesulfonic acid or cyanamide are generally performed at room temperature. Another example is the reaction of an amine with 2-methyl-1-nitroisourea in water to form a nitroguanidine. The nitro group is then easily removed to form a guanidino moiety by hydrogenolysis.

I define the term "guanidino forming agent" appearing herein to include any chemical agent capable of forming a guanidino moiety upon its reaction with a non-guanidino moiety. Examples of guanidino forming agents include S-ethylthiouronium bromide, S-ethylthiouronium chloride, O-methylisourea, O-methylisouronium sulfate, O-methylisourea hydrogen sulfate, S-methylisothiourea, 2-methyl-1-nitroisourea, aminoiminomethanesulfonic acid, cyanamide, cyanoguanide, dicyandiamide, 3,5-dimethyl-1-guanylpyrazole nitrate and 3,5-dimethyl pyrazole. In addition, the term "guanidino forming agent" appearing herein may mean any one or more of a guanidino forming agent or a combination of different guanidino forming agents.

I define the term "medical device" appearing herein as a device having surfaces that contact human or animal bodily tissue and/or fluids in the course of their operation. This definition includes within its scope, for example, extracorporeal devices for use in surgery such as blood oxygenators, blood pumps, blood sensors, tubing used to carry blood and the like which contact blood which is then returned to the patient. The definition includes within its scope endoprostheses implanted in blood contact in a human or animal body such as vascular grafts, stents, pacemaker leads, heart valves, and the like that are implanted in blood vessels or in the heart. The definition also includes within its scope devices for temporary intravascular use such as catheters, guide wires, and the like which are placed into the blood vessels or the heart for purposes of monitoring or repair.

One method of the invention may be used to modify substrates of any shape or form including tubular, sheet, rod and articles of proper shape for use in a number of medical devices such as vascular grafts, aortic grafts, arterial, venous, or vascular tubing, vascular stents, dialysis membranes, tubing or connectors, blood oxygenator tubing or membranes, ultrafiltration membranes, intra-aortic balloons, blood bags, catheters, sutures, soft or hard tissue prostheses, synthetic prostheses, prosthetic heart valves, tissue adhesives, cardiac pacemaker leads, artificial organs, endotracheal tubes, lenses for the eye such as contact or intraocular lenses, blood handling equipment, apheresis equipment, diagnostic and monitoring catheters and sensors, biosensors, dental devices, drug delivery systems, or bodily implants of any kind.

### Examples

The present invention is further described by the following examples. The examples are provided solely to illustrate the invention by reference to specific embodiments. These exemplifications, while illustrating certain specific aspects of the invention, do not portray the limitations or circumscribe the scope of the invention. Examples 2 to 7 do not form part of the invention.

### Example 1: Binding of Quinones and Catechols to Etched Silicon Rubber Surfaces

Silicon rubber (SR) surfaces were etched using KOH in methanol/water solution (12.4 g KOH, 12 ml distilled water and 100 ml methanol). Following immersion in the etching solution for 4 hours at room temperature, the etched silicon tubing was submerged at room temperature in a solution of 1.0 mM of dopamine and 1.0 mM sodium periodate in deionized (DI) water. After a 12 hour soak, the SR surface changed from white to brown, demonstrating the presence of the brown colored quinone. Subsequent observation under a microscope confirmed that the brown color, i.e. the quinone, was located on the surface of the SR. The oxidized dopamine appears to bind to etched SR under basic conditions; the preferable pH appears to be 9.

Two other catechols, hematoxylin and tiron, were bound to etched SR. Both hematoxylin and tiron were oxidized to their quinone structures using sodium periodate. These oxidized catechols bound to etched SR under basic pH as demonstrated by a color change on the surface of the SR.

### Example 2: Binding of Quinones to Polyurethane Surfaces

Pellethane 80A samples were immersed at room temperature in oxidized dopamine solutions, pH 3, 7.4, and 10. The pH 3 buffer solution contained 625 ml 0.1 M citric acid and 385 ml 0.2 M di-sodium phosphate. The pH 7.4 buffer solution was phosphate buffered saline and the pH 9 buffer solution contained 7.14 g sodium bicarbonate and 0.32 g sodium hydroxide. All of the solutions also contained 1.0 mM dopamine and 1.0 mM sodium periodate. Samples soaked in the pH 3 solution changed color from clear to yellow. Observation under a microscope demonstrated that the yellow color, i.e. the color of the quinone at pH 3, was found throughout the polyurethane material. Therefore, it appeared that the polyurethane material had absorbed the quinone. Under the conditions tested herein, oxidized dopamine appeared to absorb into the bulk of a polyurethane substrate.

### Example 3: Crosslinking PEI with Quinones

Dopamine was first oxidized using sodium periodate then put into a solution of 1% by weight polyethyleneimine (PEI), a highly branched polymer comprising amines, in deionized water. After sitting overnight at room temperature, the oxidized dopamine-PEI solution became a thick sludge. This thick sludge appeared to be-cross-linked PEI.

### Example 4: Attaching PEI with Quinones to Etched Silicon Rubber

A catechol that also possesses an aldehyde group, 2,3 dihydroxybenzaldehyde, was added to a 1% by weight PEI in deionized water solution. Next, sodium cyanoborohydride was added to the catechol-PEI solution, thereby reducing any Schiff bases formed to secondary amines. After the reaction solution sat overnight at room temperature, a pink, sludgy precipitate had formed. The precipitate was then dissolved in a pH 9 buffer solution consisting of sodium bicarbonate and sodium hydroxide, and oxidized with sodium periodate, thereby forming quinones. Etched SR samples were then immersed in the quinone-PEI solution overnight at room temperature. Following overnight immersion, a thin coating was observed on the surface of the SR material.

### Example 5: Functionalization of polyacrylamide with quinones

Solid polyurethane samples grafted with polyacrylamide were made by cutting polyurethane film samples into small squares and washing them in ethanol. Acrylamide was then surface graft polymerized to the polyurethane samples using ammonium cerium (IV) nitrate as a catalyst. The polyurethane samples were immersed while stirring for approximately 2 hours at room temperature in a grafting solution consisting of 50 wt% acrylamide in deionized water. The presence of a grafted polyacrylamide coating on the surface of the polyurethane was confirmed by FTIR/ATR. Following grafting, the samples were immersed for 3 hours at room temperature in a Hoffman rearrangement solution consisting of 20 g NaOH and 2 ml NaOCI in 178 ml of deionized water. As mentioned earlier, the Hoffman rearrangement reaction converts amides into primary amines. The amines formed on the grafted surface were then used to couple 2,3-dihydroxybenzaldehyde molecules to the derivatized polyacrylamide by immersing the grafted samples for 1 hour at room temperature in a solution consisting of excess 2,3-dihydroxybenzaldehyde in 5 ml deionized water. Following Schiff base formation, sodium cyanoborohydride was added to reduce any Schiff bases to secondary amines. Next, sodium periodate was used to oxidize the coupled 2,3-dihydroxybenzaldehydes to quinones by immersing the samples overnight in a solution consisting of excess sodium periodate in 5 ml deionized water at room temperature.

### Example 6: Functionalization of Polyvinylpyrrolidone with Quinones

Polyvinylpyrrolidone (PVP) polymers in solution were functionalized with quinones following, first, hydrolysis of the PVP and, second, a carbodiimide coupling reaction between PVP and dopamine. Attachment of dopamine molecules along the PVP chain may occur through a carbodiimide coupling reaction between the carboxylic acid groups of the hydrolyzed PVP and the amine groups of the dopamine molecules.

PVP powder (Kollidon 17PF, BASF Corp.) was first hydrolyzed in hydrochloric acid. Next, carbodiimide was added to the hydrolyzed PVP solution. Dopamine was then added, thereby replacing any carbodiimide coupled to hydrolyzed PVP polymers via a condensation reaction. Sodium periodate was then added to the solution. Following oxidation, Pellethane 80A samples were placed into the PVP-dopaquinone solution.

### Example 7: Functionalization of Polyvinylpyrrolidone on a Surface with Quinones

Pellethane 80A polyurethane film samples were cut into small squares and washed in ethanol. To achieve a PVP coated surface, N-vinylpyrrolidone was surface grafted onto the polyurethane samples using ammonium cerium (IV) nitrate as a catalyst. The polyurethane samples were immersed while stirring overnight at room temperature in a grafting solution consisting of N-vinylpyrrolidone and ammonium cerium (IV) nitrate. The presence of a relatively thin PVP coating on the surface of the samples was confirmed by FTIR/ATR. Following grafting, the PVP coating was hydrolyzed by immersing the samples in acid for approximately 3 hours at room temperature. Following hydrolysis, the samples stained a dark blue color with toluidine blue dye, indicating the presence of negatively charged carboxylic acid groups on the samples. A carbodiimide coupling reaction was then performed by placing the hydrolyzed samples in a carbodiimide (EDC) solution. The carbodiimide first coupled to the carboxylic acids formed during hydrolysis. Next, the coupled carbodiimide was replaced with dopamine through a condensation reaction. Sodium periodate was then added to the samples, thereby oxidizing the dopamine to its quinone structure.

It will be appreciated by those skilled in the art that while the invention has been described above in connection with particular embodiments and examples, the invention is not necessarily so limited, and that numerous other embodiments, examples, uses, modifications and departures from the embodiments, examples and uses are intended to be encompassed by the claims attached hereto.

## Claims

1. A process of coating a hydrophilic polymer on a surface of a medical device, **characterised in that** one of said medical device surface and said hydrophilic polymer comprises a catechol moiety and the other comprises a chemical moiety capable of forming a hydrogen bond with said catechol moiety, whereby a chemical bond is formed between said catechol moiety and said chemical moiety.

2. A process as claimed in claim 1 wherein said chemical moiety capable of forming a hydrogen bond with said catechol moiety is a hydroxyl, phosphate, sulphate, carboxylate, amide, guanidino or amine moiety.

3. A process as claimed in claim 1 or claim 2 wherein said medical device is a blood-contacting medical device, a tissue-contacting medical device, a bodily fluid-contacting medical device, an implantable medical device, an extracorporeal medical device, a blood oxygenator, a blood pump, a blood sensor, tubing for carrying blood, an endoprosthesis medical device, a vascular graft, a stent, a pacemaker lead, a heart valve, a temporary intravascular medical device, a catheter or a guide wire.

4. A process as claimed in any of the preceding claims wherein at least a portion of the medical device surface takes the form of a tube, rod, membrane, balloon, bag or sheet.

5. A process as claimed in any of the preceding claims wherein the medical device surface comprises at least one biocompatible material selected from metals (including titanium, titanium alloys, tin-nickel alloys, shape memory alloys, aluminium oxides, platinum, platinum alloys, stainless steel [including MP35N stainless steel], elgiloys and stellites; pyrolitic, silver, glassy and compressed carbons; polymers (including polyamides, polycarbonates, polyethers, polyesters, polyolefins [including polyethelenes and polypropylenes], polystyrenes, polyurethanes, polyvinyl chlorides, polyvinyl pyrrolidones, silicone elastomers, fluoropolymers [including polytetrafluoroethylenes], polyacrylates, polyisoprenes and rubbers; ceramics; hydroxyapatites; human and animal proteins and tissues; bones; skin; teeth; collagens; laminins; elastins; fibrins; wood; celluloses and glass.

6. A process as claimed in any of the preceding claims wherein the hydrophilic polymer is a naturally occurring or synthetic water-soluble or water-swellable polymer comprising a hydrophilic chemical moiety.

7. A process as claimed in claim 6 wherein the polymer is capable of reducing friction on a surface.

8. A process as claimed in claim 6 or claim 7 wherein the polymer is selected from polyacrylamides, polymethacrylamides, poly(2-acrylamido-2-methylpropane sulphonic acids), polyacrylic acids, poly[N-(3-aminopropyl)methacrylamide hydrochlorides], polyvinyl pyrrolidones, polyethylene oxides, polysaccharides (including agarose and polyglycans such as hyaluronic acid and chondroitin sulphate polymers), poly(alkylene oxalates), polyvinyl alcohols, polyionenes, polycaprolactones, cellulosic polymers (including chitins) and poly(maleic anhydrides).

9. A process as claimed in any of claims 6 to 8 wherein the polymer has a molecular weight between 100,000 and 2,000,000.

10. A process as claimed in any of the preceding claims wherein the surface of the medical device comprises a primer through which it is bound or becomes bound to the hydrophilic polymer.

11. A process as claimed in any of the preceding claims wherein any amine chemical moiety is formed by combining an amine-forming agent with an amide moiety.

12. A process as claimed in claim 11 wherein said amine-forming agent is selected from bromine, bromides, bromites, hypobromites, chlorine, chlorides, chlorites, hypochlorites, lead tetraacetate, benzyltrimethylammonium tribromide, [bis(trifluoroacetoxy)iodo]benzene, hydroxy(tosyloxy)iodobenzene and iodosylbenzene.

13. A process as claimed in any of the preceding claims wherein any guanidino chemical moiety is formed by combining a guanidino-forming agent with an amine moiety.

14. A process as claimed in claim 13 wherein the guanidino-forming agent is selected from S-ethylthiouronium bromide, S-ethylthiouronium chloride, O-methylisourea, O-methylisouronium sulphate, O-methylisourea hydrogen sulphate, S-methylisothiourea, 2-methyl-1-nitroisourea, aminoiminomethanesulphonic acid, cyanamide, cyanoguanide, dicyandiamide, 3,5-dimethyl-1-guanylpyrazole nitrate and 3,5-dimethyl pyrazole.

15. A coated medical device comprising either (a) a catechol moiety disposed on the surface of the medical device and chemically bonded to a hydrophilic polymer or (b) a hydrophilic polymer comprising a catechol moiety chemically bonded to the surface of the medical device, wherein the hydrophilic polymer is optionally bound to a primer on the surface of the medical device.

## Patentansprüche

1. Ein Verfahren zum Beschichten einer Oberfläche eines Medizinprodukts mit einem hydrophilen Polymer, **dadurch gekennzeichnet, dass** eines aus der Oberfläche des Medizinprodukts und dem hydrophilen Polymer eine Catecholgruppe umfasst und das andere eine chemische Gruppe umfasst, die in der Lage ist, eine Wasserstoffbrückenbindung mit der Catecholgruppe auszubilden, wodurch eine chemische Bindung zwischen der Catecholgruppe und der chemischen Gruppe ausgebildet wird.

2. Ein Verfahren nach Anspruch 1, wobei die chemische Gruppe, die in der Lage ist, eine Wasserstoffbrückenbindung mit der Catecholgruppe auszubilden, eine Hydroxyl-, Phosphat-, Sulfat-, Carboxylat-, Amid-, Guanidino- oder Aminogruppe ist.

3. Ein Verfahren nach Anspruch 1 oder Anspruch 2, wobei das Medizinprodukt ein mit Blut in Berührung kommendes Medizinprodukt, ein mit Gewebe in Berührung kommendes Medizinprodukt, ein mit Körperflüssigkeit in Berührung kommendes Medizinprodukt, ein implantierbares Medizinprodukt, ein extrakorporales Medizinprodukt, ein Blut-Oxygenator, eine Blutpumpe, ein Blutsensor, eine blutführende Rohrleitung, ein endprothetisches Medizinprodukt, ein Gefäßersatz, ein Stent, eine Herzschrittmacherleitung, eine Herzklappe, ein temporäres intravaskuläres Medizinprodukt, ein Katheter oder ein Führungsdraht ist.

4. Ein Verfahren nach einem der vorstehenden Ansprüche, wobei mindestens ein Teil der Oberfläche des Medizinprodukts die Form einer Röhre, eines Stabs, einer Membran, eines Ballons, einer Tasche oder einer Folie hat.

5. Ein Verfahren nach einem der vorstehenden Ansprüche, wobei die Oberfläche des Medizinprodukts mindestens ein biokompatibles Material umfasst, das aus Metallen (einschließlich Titan, Titanlegierungen, Titan-Nickel-Legierungen, Formgedächtnislegierungen, Aluminiumoxide, Platin, Platinlegierungen, rostfreier Stahl [einschließlich MP35N rostfreier Stahl], Elgiloys und Stelliten); pyrolytischen, Silber-, glasigen und verdichteten Kohlenstoffen; Polymeren (einschließlich Polyamide, Polycarbonate, Polyether, Polyester, Polyolefine [einschließlich Polyethylene und Polypropylene], Polystyrole, Polyurethane, Polyvinylchloride, Polyvinylpyrrolidone, Silicon-Elastomere, Fluorpolymere [einschließlich Polytetrafluorethylene], Polyacrylate, Polyisoprene und Gummis); Keramiken, Hydroxylapatiten; menschlichen und tierischen Proteinen und Geweben; Knochen; Haut; Zähnen; Collagenen; Lamininen; Elastinen; Fibrinen; Hölzern; Cellulosen und Gläsern ausgewählt ist.

6. Ein Verfahren nach einem der vorstehenden Ansprüche, wobei das hydrophile Polymer ein natürlich vorkommendes oder synthetisches wasserlösliches oder in Wasser quellbares Polymer ist, das eine hydrophile Gruppe umfasst.

7. Ein Verfahren nach Anspruch 6, wobei das Polymer in der Lage ist, die Reibung auf einer Oberfläche zu reduzieren.

8. Ein Verfahren nach Anspruch 6 oder Anspruch 7, wobei das Polymer aus Polyacrylamiden, Polymethacrylamiden, Poly-(2-acrylamido-2-methylpropansulfonsäuren), Polyacrylsäuren, Poly-[N-(3-aminopropyl)-methacrylamid-hydrochloriden], Polyvinylpyrrolidonen, Polyethylenoxiden, Polysacchariden (einschließlich Agarose und Polyglycanen, wie Hyaluronsäure- und Chondroitinsulfat-Polymere), Poly-(alkylenoxalaten), Polyvinylalkoholen, Polyionenen Polycaprolactonen, cellulosischen Polymeren (einschließlich Chitine) und Poly-(maleinsäureanhydriden) ausgewählt ist.

9. Ein Verfahren nach einem der Ansprüche 6 bis 8, wobei das Polymer ein Molekulargewicht zwischen 100.000 und 2.000.000 besitzt.

10. Ein Verfahren nach einem der vorstehenden Ansprüche, wobei die Oberfläche des Medizinprodukts einen Primer umfasst, durch den diese an das hydrophile Polymer gebunden ist oder gebunden wird.

11. Ein Verfahren nach einem der vorstehenden Ansprüche, wobei eine beliebige chemische Amingruppe durch Zusammenbringen eines Aminbildenden Mittels mit einer Amidgruppe gebildet wird.

12. Ein Verfahren nach Anspruch 11, wobei das Amin-bildende Mittel aus Brom, Bromiden, Bromiten, Hypobromiten, Chlor, Chloriden, Chloriten, Hypochloriten, Bleitetraacetat, Benzyltrimethylammoniumtribromid, [Bis(trifluoracetoxy)iod]benzol, Hydroxy(tosyloxy)iodbenzol und lodosylbenzol ausgewählt ist.

13. Ein Verfahren nach einem der vorstehenden Ansprüche, wobei eine beliebige chemische Guanidinogruppe durch Zusammenbringen eines Guanidino-bildenden Mittels mit einer Amingruppe gebildet wird.

14. Ein Verfahren nach Anspruch 13, wobei das Guanidino-bildende Mittel aus S-Ethylthioharnstoffbromid, S-Ethylthioharnstoffchlorid, O-Methylisoharnstoff, O-Methylisoharnstoffsulfat, O-Methylisoharnstoffhydrogensulfat, S-Methylisothioharnstoff, 2-Methyl-1-nitroisoharnstoff, Aminoiminomethansulfonsäure, Cyanamid, Cyanoguanidin, Dicyandiamid, 3,5-Dimethyl-1-guanylpyrazolnitrat und 3,5-Dimethylpyrazol ausgewählt ist.

15. Ein beschichtetes Medizinprodukt, das entweder (a) eine Catecholgruppe, die auf der Oberfläche des Medizinprodukts angeordnet ist und chemisch an ein hydrophiles Polymer gebunden ist, oder (b) ein hydrophiles Polymer umfasst, das eine Catecholgruppe umfasst, die an der Oberfläche des Medizinprodukts chemisch gebunden ist, wobei das hydrophile Polymer wahlweise an einen Primer auf der Oberfläche des Medizinprodukts gebunden ist.

## Revendications

1. Procédé d'application d'un polymère hydrophile en revêtement sur une surface d'un dispositif médical, **caractérisé en ce que** l'un de ladite surface de dispositif médical et dudit polymère hydrophile comprend une fraction catéchol et l'autre comprend une fraction chimique capable de former une liaison hydrogène avec ladite fraction catéchol, ce par quoi une liaison chimique est formée entre ladite fraction catéchol et ladite fraction chimique.

2. Procédé selon la revendication 1, dans lequel ladite fraction chimique capable de former une liaison hydrogène avec ladite fraction catéchol est une fraction hydroxyle, phosphate, sulfate, carboxylate, amide, guanidino ou amine.

3. Procédé selon l'une des revendications 1 ou 2, dans lequel ledit dispositif médical est un dispositif médical au contact du sang, un dispositif médical au contact d'un tissu, un dispositif médical au contact d'un fluide corporel, un dispositif médical implantable, un dispositif médical extracorporel, un oxygénateur sanguin, une pompe sanguine, un capteur sanguin, un tube de transport de sang, un dispositif médical endoprothétique, une greffe vasculaire, une endoprothèse, un fil de stimulateur cardiaque, une valve cardiaque, un dispositif médical intravasculaire temporaire, un cathéter ou un fil guide.

4. Procédé selon l'une quelconque des revendications précédentes, dans lequel au moins une partie de la surface du dispositif médical prend la forme d'un tube, d'une tige, d'une membrane, d'un ballonnet, d'un sac ou d'une feuille.

5. Procédé selon l'une quelconque des revendications précédentes, dans lequel la surface du dispositif médical comprend au moins un matériau biocompatible choisi parmi des métaux (comprenant le titane, les alliages de titane, les alliages étain-nickel, les alliages à mémoire de forme, les oxydes d'aluminium, le platine, les alliages de platine, l'acier inoxydable [comprenant l'acier inoxydable MP35N], les Elgiloys et les stellites ; les carbones pyrolytiques, argent, vitreux et comprimés ; les polymères (comprenant les polyamides, les polycarbonates, les polyéthers, les polyesters, les polyoléfines [comprenant les polyéthylènes et les polypropylènes], les polystyrènes, les polyuréthanes, les poly(chlorures de vinyle), les polyvinyl pyrrolidones, les élastomères de silicone, les polymères fluorés [comprenant les polytétrafluoroéthylènes], les polyacrylates, les polyisoprènes et les caoutchoucs ; les céramiques ; les hydroxyapatites ; les protéines et tissus humains et animaux ; les os ; la peau ; les dents ; les collagènes ; les laminines ; les élastines ; les fibrines ; le bois ; les celluloses et le verre.

6. Procédé selon l'une quelconque des revendications précédentes, dans lequel le polymère hydrophile est un polymère d'origine naturelle ou synthétique soluble dans l'eau ou gonflable dans l'eau comprenant une fraction chimique hydrophile.

7. Procédé selon la revendication 6, dans lequel le polymère est capable de réduire le frottement sur une surface.

8. Procédé selon l'une des revendications 6 ou 7, dans lequel le polymère est choisi parmi les polyacrylamides, les polyméthacrylamides, les poly(acides 2-acrylamido-2-méthylpropane sulfoniques), les acides polyacryliques, les poly[chlorhydrates de N-(3-aminopropyl)méthacrylamide], les polyvinyl pyrrolidones, les poly(oxydes d'éthylène), les polysaccharides (comprenant l'agarose et les polyglycanes tels que l'acide hyaluronique et les polymères de sulfate de chondroïtine), les poly(oxalates d'alkylène), les alcools polyvinyliques, les polyionènes, les polycaprolactones, les polymères cellulosiques (comprenant les chitines) et les poly(anhydrides maléiques).

9. Procédé selon l'une des revendications 6 à 8, dans lequel le polymère a une masse moléculaire entre 100 000 et 2 000 000.

10. Procédé selon l'une quelconque des revendications précédentes, dans lequel la surface du dispositif médical comprend un primaire par l'intermédiaire duquel il est lié ou devient lié au polymère hydrophile.

11. Procédé selon l'une des revendications précédentes, dans lequel toute fraction chimique amine est formée par la combinaison d'un agent de formation d'amine avec une fraction amide.

12. Procédé selon la revendication 11 dans lequel ledit agent de formation d'amine est choisi parmi le brome, les bromures, les bromites, les hypobromites, le chlore, les chlorures, les chlorites, les hypochlorites, le tétraacétate de plomb, le tribromure de benzyltriméthylammonium, le [bis(trifluoroacétoxy)iodo]benzène, l'hydroxy(tosyloxy)iodobenzène et l'iodosylbenzène.

13. Procédé selon l'une quelconque des revendications précédentes dans lequel toute fraction chimique guanidino est formée par la combinaison d'un agent de formation de guanidino avec une fraction amine.

14. Procédé selon la revendication 13, dans lequel l'agent de formation de guanidino est choisi parmi le bromure de S-éthylthiouronium, le chlorure de S-éthylthiouronium, la O-méthylisourée, le sulfate de O-méthylisouronium, l'hydrogénosulfate de O-méthylisourée, la S-méthylisothiourée, la 2-méthyl-1-nitroisourée, l'acide aminoiminométhane sulfonique, le cyanamide, la cyanoguanide, le dicyandiamide, le nitrate de 3,5-diméthyl-1-guanylpyrazole et le 3,5-diméthyl pyrazole.

15. Dispositif médical revêtu comprenant soit (a) une fraction catéchol disposée sur la surface du dispositif médical et chimiquement liée à un polymère hydrophile soit (b) un polymère hydrophile comprenant une fraction catéchol chimiquement liée à la surface du dispositif médical, le polymère hydrophile étant facultativement lié à un primaire sur la surface du dispositif médical.
